# DEMANDE DE BREVET EUROPEEN

(11) **EP 1 027 872 A1**
(43) Date de publication de la demande: **16.08.2000**
(21) Numéro de dépôt: 00830003.0
(22) Date de dépôt: 07.01.2000
(51) Int. Cl.: A61F 6/06

(54) **Slip hygiénique féminin avec préservatif en latex**

(30) Priorité: 07.01.1999 IT RG990001
(71) Demandeur: Zacco, Vincenzo, 96012 Avola (SR) Sicilia (IT)
(72) Inventeur: Zacco, Vincenzo, 96012 Avola (SR) Sicilia (IT)

(57) **Abrégé**

SLIP PRÉSERVATIF FEMININ avec réservoir et chàssis de latex ou en synthétique résine contre l'allergie extrasensible protecteur contre l'AIDS, les grossesses pas désirées et pour le déblocage des psychoses de l'homme impuissant faut de l'érection du pénis à cause peur de l'AIDS et répugnance du Préservatif Masculin qui lui est demandé à mettre. Le SLIP Préservatif Feminin avant le rapport charnel le met la femme, aprés le caresses intimes, avec le réservoir en l'entrée du vagin, pour l'hygiéne avec un doigt de gomme. Aprés le rapport la femme enlève le Slip et avec lui sort le réservoir du vagin. L'homme a eu son rapport sans mettre le préservatif masculin et la sûreté 100/100 de ne pas être infecté de l'AIDS, la méme chose pour la femme. Le Slip Préservatif Feminin en latex ou en synthétique résine extrasensible assure la conductibilité de la chaleur corporelle pendant le rapport sexuel. L'usage est pratique, hygiénique et sûr de toutes les infections, de l'AIDS et derivés hépatiques A-B-C. Le SLIP sera en unique morceau USER et JETER. LA FEMME EST LA PLUS QUALIFIÉE POUR LA DÉFENSE CONTRE L'AIDS.

## Description

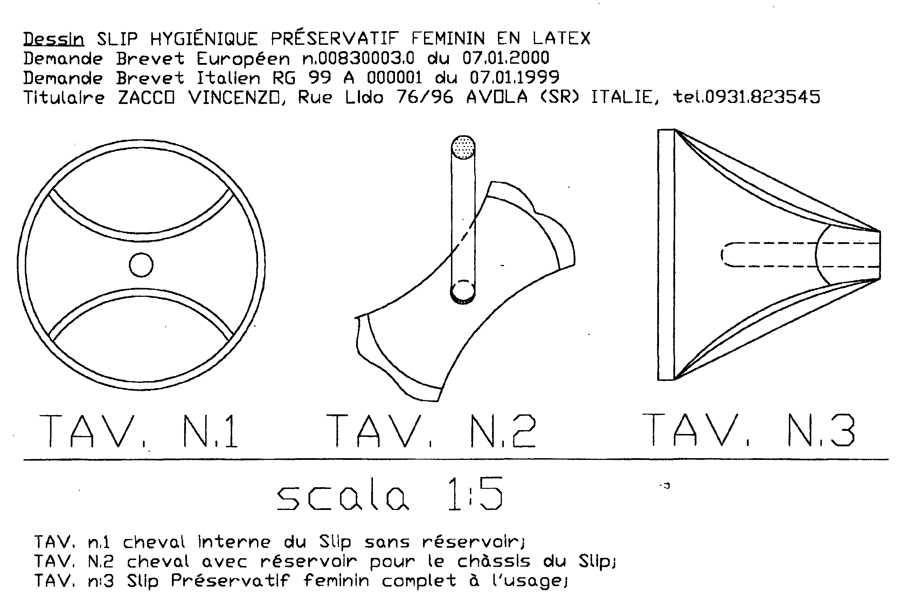

Dans le prospectus A/M le dessin n.I est le cheval interne du slip sans réservoir; N.2 est le cheval interne du slip compris le réservoir; le dessin N.3 est le slip préservatif complet que la femme devra mettre avant le rapport charnel. Le Slip produit en lactique extrasensible en unique morceau de série sera confectionné dans les tailles n.40/56 et autre requête de commerce. Le Slip autour de flancs sera achevé par un élastique comme tous les normaux slip féminins qui se trouvent en commerce. Par conséquent l'usage est pratique, hygienique et sûr à I00/I00 de toutes les infections et de l'AIDS. Le Slip à la femme est facile à l'eulever après l'usage du rapport sexuel en restant propre du coit de l'homme sans recevoir on trausmettre infections et l'AIDS. Le Slip avant le rapport charnel le mettre la femme le réservoir en l'entrée du vagin, a vouloir de hygiéne, avec un doigt de gomme. Le réservoir en lactique extrasensible sera commode comme largeur et longueur pour se mettre eu ordre dans le vagin eu évitant le direct contact charnel et eu le protegeant contre toutes les infections. Le réservoir sera lubrific à l'intérieur pour rendre plus sensible le rapport charnel entre la femme et l'homme. Le réservoir sera porté en profondeur du pénis. Le SLIP HYGIENIQUE PRESERVATIF FEMININ est absolument urgente nécessité parce que l'AIDS propage rapidement la mort de beaucoup de millions de personnes humaines adultes et enfants dans le monde, défend de toutes les maladies infectienses et vénériennes sexuelle, défend les familles avec plus enfants à leur charge et défend l'amour des jeunes-gens sans grossesses.

## Revendications

1. C'est un préservatif imaginé pour la femme en défense du genere humain du mortel AIDS, de l'impuissance des hommes et des grossesses pas désirées des filles ni des femmes mariées ni des yeunes sans marito. Sont beaucoups les hommes qui refusent l'invitation des prostitués d'employer le préservatif avec toutes les conséquence de propagation des maladies et de l'AIDS. Sont beaucoups les hommes, tants millions, à pen pres d'impuissants quise sont adaptés à leur impuissance sans comprendre que cela défaut être un facteur psychologique à cause de l'AIDS ou de répugnance à l'usage du préservatif masculin. Tout le monde est de mode la pilules et cependant le 70/100 environ ils sont gueris de leur incosciente impuissance et récupérés a leur virilité par l'usage du SLIP Féminin de part des fémmes avec assuré à 100/100 de tutes les infections et débloquant de la psychose du préservatif masculin a mettre. Certainement l'Etat devra obliger par Décret Ministériel de la Santé l'usage du SLIP Préservatif Féminin à toutes les femmes qui excercent la profession de la prostitution pour la leur défense à cause de l'AIDS sans se réfuser à l'homme client qui réfuse d'endosser le préservatif masculin pour le rapport charnel. Le réfus provoque la réaction de l'homme dangereuse à l'intégrité des prostitucés parce que l'homme client est bloquée de la psychose du préservatif masculin. La même chose c'est pour la défense des yeunes sans marito de les grossesses qui provoquent la mort de plusieurs enfants et qui les yettent dans les caisses publiques de l'immondice ou le suicide de la fille mère sans travvail ou étudiante. Le Slip Préservatif défend les femmes mariées parce que elles ne désirent pas de grossesses surtant pour leur économie familiale parce qu'elles ont deux on trois enfants à leur charge. Les pilules coûteuses qui pèsent sur la balance familiale et nationale pour les impuissants et les grossesses en même temps son dangeurenses pour le coeur et peuvent aussi causer des trumeurs à l'uterus et à l'ovaire.
Donc l'usage du SLIP Préservatif Féminin révolutionne et apporte une grande sécurité et un grand bénéfice à l'homme et à la femme eu cas de rapport sexuel et ainsi BLOQUER LA PROPAGATION DE L'AIDS avec definition du Secrétaire Général de l'ONU, MONSIEUR KOFI ANNAN, la PESTE DU 2000 POUR L'ENTIERE CIVILITÉ.
